# EUROPEAN PATENT SPECIFICATION

(11) **EP 1 037 604 B1**
(45) Date of publication and mention of the grant of the patent: **26.01.2005**
(21) Application number: 98955755.8
(22) Date of filing: 20.11.1998
(51) Int. Cl.: A61K 9/00, A61K 9/12

(54) **PHARMACEUTICAL AEROSOL COMPOSITIONS**
PHARMAZEUTISCHE AEROSOLZUSAMMENSETZUNGEN
COMPOSITIONS PHARMACEUTIQUES D'AEROSOLS

(30) Priority: 08.12.1997 GB 9725984
(43) Date of publication of application: 27.09.2000
(73) Proprietor: MINNESOTA MINING AND MANUFACTURING COMPANY, St. Paul, Minnesota 55133-3427 (US)
(72) Inventor: PRITCHARD,Yvonne,Mary,Beatrice Honeysuckle Cottage, Epperstone Nottingham NG14 6AE (GB); CLAIRE, Kanwaljit, Singh, Blaby Leicester LE8 4DL (GB)
(74) Representative: Bowman, Paul Alan
(86) International application number: PCT/GB1998/003488
(87) International publication number: WO 1999/029296

(56) References cited:
- WO-A-93/11745
- WO-A-96/18384
- WO-A-98/01147
- WO-A-99/65464
- US-A- 5 589 156

## Description

### FIELD OF THE INVENTION

This invention relates to pharmaceutical aerosol compositions and in particular to pharmaceutical aerosol compositions comprising a plurality of active ingredients.

### BACKGROUND OF THE INVENTION

The use of a self-propelling composition comprising one or more aerosol propellants as a delivery system for medicament has been known for many years. Such aerosol compositions have been used for topical applications and for delivering of medicaments to the respiratory system of a patient. Since the metered dose inhaler (MDI) was introduced in the mid 1950's, inhalation has become a widely used route for delivery of drugs for the treatment of bronchial maladies and as an alternative to other administration routes for drug delivery for treatment of maladies which are not primarily concerned with the respiratory system.

MDI formulations generally comprise one or more propellants, drug which is dissolved or suspended in the propellant and a surfactant to aid in dissolution or suspension of the drug in propellant, to act as a lubricant for the valve and/or stabilise the formulation. The formulations often comprise a further adjuvant, such as a cosolvent, to solubilise the surfactant and/or drug in the propellant. It is important that the drug be either readily soluble or substantially insoluble in the formulations. Partial dissolution can result in problems with crystal growth and for inhalation therapy particles of from 1 to 10µm are required for penetration into the bronchioles or pulmonary regions of the lung.

Most of the medicinal aerosol formulations which are available contain a single active ingredient. However, some "compound" preparations containing two or more drugs are known. The following are examples of marketed products containing two or more drugs in suspension formulations. The aerosol formulation commercially available under the trade name Combivent from Boehringer Ingelheim comprises a mixture of ipratropium bromide and salbutamol sulphate.

The aerosol formulation commercially available under the trade name Duovent from Boehringer Ingelheim comprises a mixture of fenoterol hydrobromide and ipratropium bromide.

The aerosol formulation commercially available under the trade name Ventide from Allen & Hanburys Limited comprises a mixture of beclomethasone dipropionate and salbutamol.

The aerosol formulation commercially available under the trade name Aerocrom from Fisons comprises a mixture of disodium cromoglycate and salbutamol sulphate.

The aerosol formulation commercially available under the trade name Aarane from Fisons Arzneimittel GmbH comprises a mixture of disodium cromoglycate and reproterol hydrochloride.

The aerosol formulation commercially available under the trade name Tobispray from Boehringer Ingelheim comprises a mixture of tramazoline hydrochloride and dexamethasone-21-isonicotinate.

The aerosol formulation commercially available under the trade name Duo-Medihaler from 3M UK plc comprises a mixture of isoproterenol hydrochloride and phenylephrine bitartrate.

The aerosol formulation commercially available under the trade name Allergospasmin from Degussa Pharma Gruppe comprises a mixture of disodium cromoglycate and reproterol hydrochloride.

The aerosol formulation commercially available under the trade name Clenil Cemposistum from Chiesi Farmaceutici comprises a mixture of beclomethasone dipropionate and salbutamol.

The aerosol formulation commercially available under the trade name Berodual from Boehringer Ingelheim comprises a mixture of ipratropium bromide and fenoterol hydrobromide.

Other compound preparations are disclosed in US-A-3320125.

In all of these known compound preparations the drugs present in a preparation are in the same form i.e., they are both present as suspensions or both in solution.

WO96/18384 discloses pharmaceutical aerosol formulations comprising:
(a) 1,1,1,2-tetrafluoroethane, 1,1,1,2,3,3,3-heptafluoro-n-propane or mixtures thereof as propellant;
(b) 1,1,2,2,3-pentafluoropropane as co-propellant; and
(c) particulate medicament

The formulations may contain surfactant and an adjuvant having a higher polarity and/or a boiling point than the propellant. The formulation may comprise two or more particulate medicaments.

WO93/11745 discloses pharmaceutical aerosol formulations which comprise particulate medicament, fluorocarbon or hydrogen-containing chlorofluorocarbon propellant and up to 5% w/w based upon propellant of a polar co-solvent, the formulations being substantially free of surfactant. It is stated these formulations may contain two or more particulate medicaments. Example 26 discloses an aerosol formulation consisting of 0.132% w/w salbutamol, 0.066% w/w beclomethasone dipropionate, 2% w/w ethanol and 1,1,1,2-tetrafluoroethane. Although it is not stated, it appears that the quantity of ethanol present is sufficiently high in this example that the beclomethasone dipropipnate is completely dissolved and salbutamol suspended in the propellant.

WO98/01147 (EP-A-0914143) published 15^{th} January, 1998 discloses a solution formulation of Cyclosporin A in 1,1,1,2,3,3,3-heptafluoropropane which is suitable for administration to a patient by inhalation using any standard medicinal aerosol device. Standard excipients normally used in medicinal aerosol formulations to aid valve lubrication or improve flavour may also be added. Other medicaments in solution or suspension may be used in addition to Cyclosporin A and other propellants in addition to 1,1,1,2,3,3,3-heptafluoropropane may be used.

US-A-5 589 156 discloses an aerosol composition for delivering topical local anaesthetic to a mammal, comprising:
1-99% wt. of hydrofluorocarbon propellant selected from the group consisting of 1,1,1,2-tetrafluoroethane and 1,1,1,2,3,3,3-heptafluoropropane and combinations thereof, said hydrofluorocarbon propellant being the sole propellant in said aerosol composition;
1-99% wt. prilocaine base solubilised within said hydrofluorocarbon propellant, said prilocaine base and said hydrofluorocarbon propellant forming a solution that is stable at reduced temperature of at least -82°C; and
0.01-99% wt. of a pharmaceutical other than prilocaine solubilised in said hydrofluorocarbon propellant selected from the group consisting of bronchodilators, anti-inflammatories, anti-tusives, vasoactive drugs, vasoconstrictors, antibiotics which are not antiseptics, peptides which are not enzymes, steroids, enzymes, antihistamines, hormones, 5-aminolevulinic acid, antiseptics, disinfectants, procaine, cocaine, chloroprocaine, tetracaine, mepivacaine, lidocaine, bupivacaine, etidocaine, ropivacaine, benzocaine, and phenylephrine.

The compositions do not contain additional organic solvents and surfactants and it is stated the association or complex between prilocaine and HFC propellants is disrupted by the presence of water or ethanol.

It has now been found that stable aerosol formulations may be prepared comprising two drugs, one of which is in suspension and the other in solution.

### BRIEF SUMMARY OF THE INVENTION

Therefore according to the present invention there is provided a medicinal aerosol formulation comprising propellant, ethanol, particles of a first drug suspended in the propellant and a second drug completely dissolved in the aerosol formulation
wherein when the first dug is salbutamol or a salt thereof and the second drug is beclomethasone dipropionate, the formulation comprises from 2 to 5 mg/ml salbutamol sulphate, 1 to 5 mg/ml beclomethasone dipropionate, from 4 to 15 % by weight ethanol and propellant selected from P134a, P227 and mixtures thereof, with the proviso that the formulation does not comprise a solution formulation of Cyclosporin A in 1,1,1,2,3,3,3-heptafluoropropane (P227).

Surprisingly, it has been found that by suitable selection of the components the presence of dissolved drug does not deleteriously affect the stability of the suspended drug particles.

### DESCRIPTION OF PREFERRED EMBODIMENTS

The propellant used in the formulation is preferably a non-CFC propellant e.g. a hydrofluoroalkane (HFA). The most preferred propellants are P134a and P227 which can be used alone or in admixture.

In principle any drug which can be rendered soluble or substantially insoluble in the aerosol formulation may be used in the formulations of the invention. The combination of drugs will be selected for the therapeutic effect. Examples of suitable drugs include antiallergics, analgesics, bronchodilators, antihistamines, therapeutic proteins and peptides, antitussives, anginal preparations, antibiotics, antiinflammatory preparations, hormones, or sulfonamides, such as, for example, a vasoconstrictive amine, an enzyme, an alkaloid or a steroid, and synergistic combinations of these specific examples or drugs which may be employed are: isoproterenol [alpha-(isopropylaminomethyl) protocatechuyl alcohol], phenylephrine, phenylpropanolamine, glucagon, adrenochrome, trypsin, epinephrine, ephedrine, narcotine, codeine, atropine, heparin, morphine, dihydromorphinone, ergotamine, scopolamine, methapyrilene, cyanocobalamin, terbutaline, rimiterol, salbutamol, formoterol, salmeterol, budesonide, isoprenaline, fenoterol, ipratropium bromide, oxitropium bromide, reproterol, fluticasone propionate, flunisolide, triamcinolone acetonide, mometasonefuroate, colchicine, pirbuterol, beclomethasone dipropionate, orciprenaline, fentanyl, and diamorphine. Others are antibiotics, such as neomycin, streptomycin, penicillin, procaine penicillin, tetracycline, chlorotetracycline and hydroxytetracycline; adrenocorticotropic hormone and adrenocortical hormones, such as cortisone, hydrocortisone, hydrocortisone acetate and prednisolone; antiallergy compounds such as cromolyn sodium, nedocromil, protein and peptide molecules such as insulin, pentamidine, calcitonin, amiloride, interferon, LHRH analogues, DNAase, heparin, etc.

The drugs exemplified above may be used as either the free base or as one or more salts known to the art. The choice of free base or salt will be influenced by the physical stability of the drug in the formulation. For example, it has been shown that the free base of salbutamol exhibits a greater dispersion stability than salbutamol sulphate in the aerosol formulations.

The following salts of the drugs mentioned above may be used; acetate, benzenesulphonate, benzoate, bicarbonate, bitartrate, bromide, calcium edetate, camsylate, carbonate, chloride, citrate, dihydrochloride, edetate, edisylate, estolate, esylate, fumarate, fluceptate, gluconate, glutamate, glycollylarsanilate, hexyiresorcinate, hydrobromide, hydrochloride, hydroxynaphthoate, iodide, isethionate, lactate, lactobionate, malate, maleate, mandelate, mesylate, methylbromide, methylnitrate, methylsulphate, mucate, napsylate, nitrate, pamoate (embonate), panthothenate, phosphate/diphosphate, polygalacturonate, salicylate, stearate, subacetate, succinate, sulphate, tannate, tartrate, triethiodide and xinafoate.

Cationic salts may also be used. Suitable cationic salts include the alkali metals, e.g., sodium and potassium, and ammonium salts and salts of amines known in the art to be pharmaceutically acceptable, e.g., glycine, ethylene diamine, choline, diethanolamine, triethanolamine, octadecylamine, diethylamine, triethylamine, 1-amino-2-propanol-amino-2-(hydroxymethyl)propane-1-3-diol and 1-(3,4-dihydroxyphenyl)-2 isopropylaminoethanol.

For pharmaceutical purposes the particle size of the powder for the drug dispersion should desirably be no greater than 100 µm (microns) diameter, since larger particles may clog the valve or orifice of the container. Preferably the particle size should be less than 25 µm (microns) in diameter. Desirably the particle size of the finely-divided solid powder should for physiological reasons be less than 25 µm (microns) and preferably less than about 10 µm (microns) in diameter. The particle size of the powder for inhalation therapy should preferably be in the range 2 to 10 µm (microns). There is no lower limit on particle size except that imposed by the use to which the aerosol produced is to be put.

The concentration of each medicament depends upon the desired dosage but is generally in the range 0.01 to 5% by weight.

The suspended drug should be insoluble in the aerosol formulation. Partial solubility of drug is undesirable since it can result in crystal growth due to Ostwald Ripening. The dissolved drug should be completely soluble in the aerosol formulation over the temperature range likely to be encountered by the product during storage and use.

The formulation of the invention comprises ethanol, which is generally present in an amount of from 2 to 20% by weight of the formulation.

The formulations may comprise surfactant. Suitable surfactants for aerosol formulations are well known and disclosed, for example, in EPOA-0372777. Preferred surfactants include oleic acid and sorbitan trioleate. Preferably the formulations are free of surfactant.

The propellant is preferably selected from P134a, P227 and mixtures thereof. Variation of the concentration of P134a and P227 in mixtures allows adjustment of the density of the aerosol formulation to match the density of the suspended drug. Density matching may decrease the rate of sedimentation or creaming of the suspending particles. Variation of concentration of any adjuvant may also contribute to density matching.

Suitable drugs which may be readily dissolved in the aerosol formulations include beclomethasone dipropionate and budesonide. These drugs may be completely dissolved in the formulations in therapeutic amounts e.g. 1 mg/ml in the presence of relatively low concentration of ethanol e.g. 8% by weight or less. Drugs such as flunisolide and butixocort propionate require higher therapeutic amounts e.g. 6 mg/ml and consequently may need larger amounts of ethanol to prepare a stable formulation.

Preferred drugs used in suspension include salbutamol base, salbutamol sulphate, terbutaline sulphate and pirbuterol acetate.

A particularly preferred formulation comprises p134a and/or p227, salbutamol sulphate in suspension, beclomethasone dipropionate in solution and sufficient ethanol to maintain the latter in solution. The formulation is preferably free of surfactant.

Generally the salbutamol sulphate will be present in a concentration of from 2 to 5 mg/ml and beclomethasone dipropionate will be present in a concentration of from 1 to 5 mg/ml and the formulation will comprise from 4 to 15% by weight ethanol, preferably about 8% by weight ethanol.

The invention will now be illustrated with reference to the following Examples.

### Examples 1 to 21

The formulations reported in the following Table 1 were prepared by addition to the beclomethasone dipropionate and salbutamol sulphate to a trimline can (a 100 ml container). The ethanol was then added, and homogenised using a Silverson mixer for five minutes. A non-metering valve was then crimped onto the can, and the propellant (p134a or p227) was added. The formulation was then chilled down in a cryobath and transferred after removing the valve to 10 ml aluminium vials and 10 ml PET bottles, which were sealed with suitable valves.

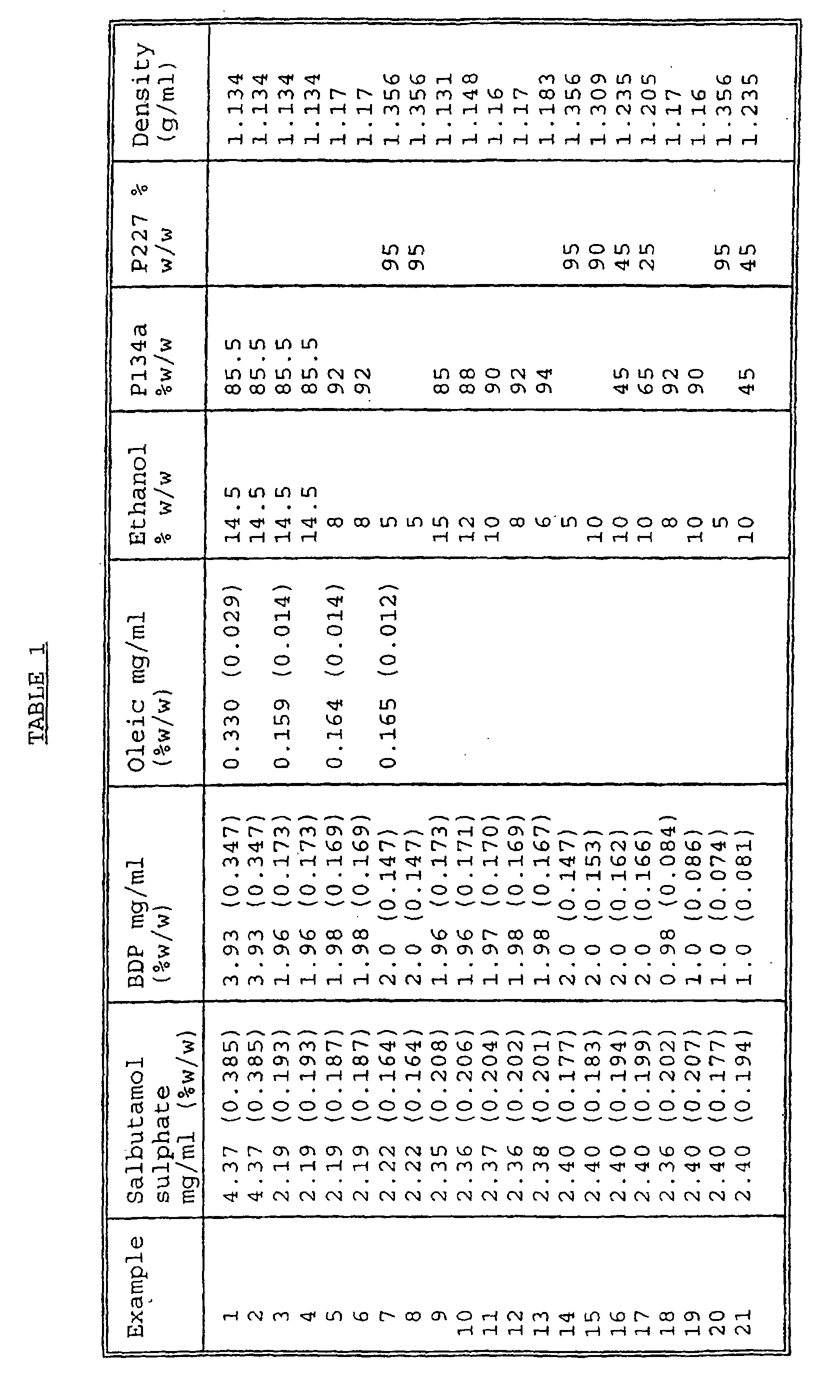

The flocculation and sedimentation/creaming rates were assessed visually by means of time lapse photography.

### Examples 1 to 8

Examples 1 and 2 flocculated at the quickest rate due to the larger concentrations of drug present.

Examples 7 and 8 formed floccules at the slowest rate since the formulations are substantially density matched. The flocculation rates of Examples 3 to 6 were similar, with Examples 5 and 6 flocculating slightly quicker than Examples 3 and 4.

Examples 1 to 8 all appeared as a white suspension; Examples 1 to 6 gradually sedimented and Examples 7 and 8 gradually creamed.

After standing for 3 days all formulations resuspended easily upon gentle agitation with no agglomerates or deposits sticking to the bottom of the vial.

### Examples 9 to 13

All formulations sedimented. It was observed there was a trend in increasing rate of sedimentation as the ethanol concentration decreases.

### Example 14

The drug flocculates and stays in suspension for about 1.5 minutes during which it begins to cream to the top. After 5 minutes larger floccules have formed which very slowly float to the top.

### Example 15

The drug flocculates and stays in suspension for about 1 minute, after which large floccules begin to form. Sedimentation of the drug is very slow and has not started after 5 minutes. After 25 minutes very large floccules have formed, some of the drug is still in suspension with sedimentation starting to occur.

### Example 16

The drug flocculates and begins to form large floccules as it simultaneously sediments. Sedimentation is apparent after 1 minute and clearly observed after 2 minutes.

### Example 17

The drug flocculates and forms large floccules as it simultaneously sediments. Sedimentation begins after 30 seconds and is clearly observed after 1 minute. After 2 minutes nearly all the drug has settle to the bottom. The rate of flocculation and sedimentation of Example 17 is very similar and visually indistinguishable from that of Example 16.

### Example 18

The drug flocculates and simultaneously sediments within a minute. After 2 minutes most of the drug has sedimented with a small amount still in suspension.

### Example 19

The drug flocculates and simultaneously sediments after about 30 seconds. After 2 minutes most of the drug has sedimented. The sedimentation process was very similar to that of Example 18. The sedimentation rate for Example 19 which contains 10% EtOH, is slightly quicker than that of Example 18, which contains 8% EtOH. The density of Example 18 (1170.0 mg/ml) is more closely matched to that of Salbutamol sulphate than the density of Example 19 (1160.0 mg/ml).

### Example 20

The drug flocculates after 30 seconds and creaming starts. After 1 minute floccules are becoming bigger and creaming is occurring slowly. After 3.5 minutes creaming is clearly observed but some of the drug is still in suspension. After 15 minutes most of the drug has creamed to the top. The observations made are comparable and similar to those made on formulation Example 14 which has the same propellant system and density as Example 20. However, Example 14 (which has a greater BDP content of 2 mg/ml) takes slightly longer to cream, some of the drug still being in suspension after 30 minutes.

### Example 21

The drug flocculates and simultaneously sediments after about 30 seconds. After 1 minute large floccules have formed and sedimentation is clearly taking place. After 2 minutes most of the drug has sedimented. The observations made are comparable and similar to those of formulation Example 16 which has the same propellant system and density. The sedimentation rate of Example 16 (BDP content 2 mg/ml) is quicker than that of Example 21 (BDP content of 1 mg/ml).

### Examples 22 to 26

The formulation reported in the following Table 2 were prepared.

**Table 2**

| | Example | | | |
|---|---|---|---|---|
| mg/ml | 22 | 23 | 24 | 25 |
| Salbutamol Sulphate | - | - | - | 2.40 |
| BDP | 2.00 | 2.00 | 1.00 | - |
| Terbutaline sulphate | 5.00 | - | - | - |
| Salbutamol Base | - | 2.00 | - | - |
| Pirbuterol Acetate | - | - | 6.30 | - |
| Budesonide | - | - | - | 1.00 |
| Ethanol (8%) | 93.040 | 93.280 | 93.016 | 93.328 |
| P134a (92%) | 1069.960 | 1072.720 | 1069.684 | 1073.272 |
| Total | 1170.00 | 1170.00 | 1170.00 | 1170.00 |

All the formulations were prepared using the method described previously with the exception of Example 25 which was homogenised using the Silverson mixer for the period of 10 minutes rather than the stated 5.

Each of the formulations were shaken in clear, plastic vials to give an even, white homogeneous suspension of drug.

### Example 22

On shaking the drug flocculates and begins to form large floccules as it simultaneously sediments. Sedimentation is apparent after approximately 30 seconds and clearly observed at around 1 minute.

### Example 23

On shaking the drug flocculates and begins to form large floccules and creaming begins. After 1 minute the floccules are becoming larger and are slowly rising to the surface. Creaming can be clearly observed after approximately 10 minutes. Complete creaming occurs after approximately 30 minutes.

### Example 24

On shaking the drug flocculates and begins to form large floccules as it simultaneously sediments. Sedimentation is apparent after approximately 20 seconds and clearly observed at around 1 minute.

### Example 25

On shaking the drug flocculates as it simultaneously sediments. Sedimentation is apparent after approximately 30 seconds and clearly observed at around 2 minutes. The vast majority of the suspended drug sediments within 3 minutes leaving a small quantity of drug still in suspension which eventually sediments after approximately 45 minutes.

## Claims

1. A medicinal aerosol formulation comprising propellant, ethanol, particles of a first drug suspended in the propellant and a second drug completely dissolved in the aerosol formulation
wherein when the first dug is salbutamol or a salt thereof and the second drug is beclomethasone dipropionate, the formulation comprises from 2 to 5 mg/ml salbutamol sulphate, 1 to 5 mg/ml beclomethasone dipropionate, from 4 to 15 % by weight ethanol and propellant selected from P134a, P227 and mixtures thereof, with the proviso that the formulation does not comprise a solution formulation of Cyclosporin A in 1,1,1,2,3,3,3-heptafluoropropane (P227).

2. A medicinal aerosol formulation as claimed in Claim 1 in which the propellant is free from CFC propellants.

3. A medicinal aerosol formulation as claimed in Claim 1 or Claim 2 in which the propellant comprises one or more hydrofluoroalkanes.

4. A medicinal aerosol formulation as claimed in any preceding Claim in which the propellant is selected from P134a, P227 and mixtures thereof.

5. A medicinal aerosol formulation as claimed in any preceding Claim which comprises a surfactant.

6. A medicinal aerosol formulation as claimed in Claim 5 in which the surfactant is selected from sorbitan trioleate and oleic acid.

7. A medicinal aerosol formulation as claimed in any one of Claims 1 to 4 which is free of surfactant.

8. A medicinal aerosol formulation as claimed in any preceding Claim in which the first drug is selected from salbutamol, terbutaline, pirbuterol and salts thereof.

9. A medicinal aerosol formulation as claimed in any preceding Claim in which the second drug is selected from beclomethasone dipropionate and budesonide.

10. A medicinal aerosol formulation as claimed in any preceding Claim in which the first drug is salbutamol or a salt thereof and the second drug is beclomethasone dipropionate.

11. A medicinal aerosol formulation as claimed in Claim 10 which comprises about 8% by weight ethanol.

12. A medicinal product comprising an aerosol container equipped with a dispensing valve, and containing an aerosol formulation as claimed in any preceding Claim.

## Patentansprüche

1. Medizinische Aerosolformulierung, umfassend ein Treibmittel, Ethanol, Teilchen eines ersten Arzneistoffs, der in dem Treibmittel suspendiert ist, und einen zweiten Arzneistoff, der vollständig in der Aerosolformulierung gelöst ist,
wobei, wenn der erste Arzneistoff Salbutamol oder ein Salz davon ist, und der zweite Arzneistoff Beclomethasondipropionat ist, die Formulierung 2 bis 5 mg/ml Salbutamolsulfat, 1 bis 5 mg/ml Beclomethasondipropionat, 4 bis 15 Gew.-% Ethanol und das Treibmittel, ausgewählt aus P134a, P227 und Mischungen davon, umfasst, mit der Maßgabe, dass die Formulierung nicht eine Lösungsformulierung von Cyclosporin A in 1,1,1,2,3,3,3-Heptafluorpropan (P227) umfasst.

2. Medizinische Aerosolformulierung nach Anspruch 1, in der das Treibmittel frei von FCKW-Treibmitteln ist.

3. Medizinische Aerosolformulierung nach Anspruch 1 oder Anspruch 2, in der das Treibmittel eines oder mehrere Hydrofluoroalkane umfasst.

4. Medizinische Aerosolformulierung nach einem der vorhergehenden Ansprüche, in der das Treibmittel ausgewählt ist aus P134a, P227 und Mischungen davon.

5. Medizinische Aerosolformulierung nach einem der vorhergehenden Ansprüche, die ein Tensid umfasst.

6. Medizinische Aerosolformulierung nach Anspruch 5, in der das Tensid ausgewählt ist aus Sorbitantrioleat und Ölsäure.

7. Medizinische Aerosolformulierung nach einem der Ansprüche 1 bis 4, die frei von Tensiden ist.

8. Medizinische Aerosolformulierung nach einem der vorhergehenden Ansprüchen, in der der erste Arzneistoff ausgewählt ist aus Salbutamol, Terbutalin, Pirbuterol und Salzen davon.

9. Medizinische Aerosolformulierung nach einem der vorhergehenden Ansprüche, in der der zweite Arzneistoff ausgewählt ist aus Beclomethasondiproprionat und Budesonid.

10. Medizinische Aerosolformulierung nach einem der vorhergehenden Ansprüche, in der der erste Arzneistoff Salbutamol oder ein Salz davon ist, und der zweite Arzneistoff Beclomethasondipropionat ist.

11. Medizinische Aerosolformulierung nach Anspruch 10, die etwa 8 Gew.-% Ethanol umfasst.

12. Medizinisches Produkt, umfassend einen Aerosolbehälter, der mit einem Abgabeventil ausgestattet ist, und enthaltend eine Aerosolformulierung nach einem der vorhergehenden Ansprüche.

## Revendications

1. Formulation médicamenteuse en aérosol comprenant un agent propulseur, de l'éthanol, des particules d'un premier médicament en suspension dans l'agent propulseur, et un second médicament dissous totalement dans la formulation en aérosol,
dans laquelle, lorsque le premier médicament consiste en salbutamol ou un de ses sels et le second médicament consiste en dipropionate de béclométhasone, la formulation comprend 2 à 5 mg/ml de sulfate de salbutamol, 1 à 5 mg/ml de dipropionate de béclométhasone, 4 à 15 % en poids d'éthanol et un agent propulseur choisi entre P134a, P227 et leurs mélanges, sous réserve que la formulation ne comprenne pas une formulation de solution de cyclosporine A dans le 1,1,1,2,3,3,3-heptafluoropropane (P227).

2. Formulation médicamenteuse en aérosol suivant la revendication 1, dans laquelle l'agent propulseur est dépourvu d'agents propulseurs consistant en CFC.

3. Formulation médicamenteuse en aérosol suivant la revendication 1 ou la revendication 2, dans laquelle l'agent propulseur comprend un ou plusieurs hydrofluor-alcanes.

4. Formulation médicamenteuse en aérosol suivant l'une quelconque des revendications précédentes, dans laquelle l'agent propulseur est choisi entre le P134a, le P227 et leurs mélanges.

5. Formulation médicamenteuse en aérosol suivant l'une quelconque des revendications précédentes, qui comprend un agent tensio-actif.

6. Formulation médicamenteuse en aérosol suivant la revendication 5, dans laquelle l'agent tensio-actif est choisi entre le trioléate de sorbitanne et l'acide oléique.

7. Formulation médicamenteuse en aérosol suivant l'une quelconque des revendications 1 à 4, qui est dépourvue d'agent tensio-actif.

8. Formulation médicamenteuse en aérosol suivant l'une quelconque des revendications précédentes, dans laquelle le premier médicament est choisi entre le salbutamol, la terbutaline, le pirbutérol et leurs sels.

9. Formulation médicamenteuse en aérosol suivant l'une quelconque des revendications précédentes, dans laquelle le second médicament est choisi entre le dipropionate de béclométhasone et le budésonide.

10. Formulation médicamenteuse en aérosol suivant l'une quelconque des revendications précédentes, dans laquelle le premier médicament est le salbutamol ou un de ses sels et le second médicament est le dipropionate de béclométhasone.

11. Formulation médicamenteuse en aérosol suivant la revendication 10, qui comprend environ 8 % en poids d'éthanol.

12. Produit médicamenteux comprenant un récipient d'aérosol équipé d'une valve distributrice, et contenant une formulation en aérosol suivant l'une quelconque des revendications précédentes.
